(19) Europäisches Patentamt

European Patent Office

Office européen des brevets

(11) EP 1 907 839 B1

(12) EUROPEAN PATENT SPECIFICATION

(45) Date of publication and mention
of the grant of the patent:
**21.08.2013 Bulletin 2013/34**

(51) Int Cl.:
*G01N 33/50* (2006.01)    *G01N 33/84* (2006.01)
*G06Q 99/00* (2006.01)

(21) Application number: **06786877.8**

(86) International application number:
**PCT/US2006/026863**

(22) Date of filing: **12.07.2006**

(87) International publication number:
**WO 2007/008882 (18.01.2007 Gazette 2007/03)**

(54) **METHODS FOR PREDICTING URINE PH**

VERFAHREN ZUR VORHERSAGE DES PH-WERTS VON URIN

PROCÉDÉS DESTINÉS À ESTIMER LE PH DE L'URINE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **12.07.2005 US 698311 P**

(43) Date of publication of application:
**09.04.2008 Bulletin 2008/15**

(73) Proprietor: **Hill's Pet Nutrition, Inc.
Topeka, KS 66603 (US)**

(72) Inventor: **YAMKA, Ryan Michael
Topeka, Kansas 66610 (US)**

(74) Representative: **Daniels, Jeffrey Nicholas
Page White & Farrer
Bedford House
John Street
London WC1N 2BF (GB)**

(56) References cited:
**US-A- 5 143 023    US-A1- 2005 106 753**

- **MARKWELL, P.J., ET AL: "The Effect of Diet on
Lower Urinary Tract Diseases in Cats" THE
JOURNAL OF NUTRITION, vol. 128, 1998, pages
2753S-2575S, XP002523085**

- **KIENZLE E ET AL: "UNTERSUCHUNGEN ZUR
STRUVITSTEINDIAETETIK 1. EINFLUSS
VERSCHIEDENER FUTTERRATIONEN AUF DEN
HARN-PH-WERT DER KATZE.
ÖINVESTIGATIONS ON STRUVITE DIETARY
TREATMENT: 1. INFLUENCE OF FOOD RATIONS
ON URIN PH IN THE CAT" DEUTSCHE
TIERAERZTLICHE WOCHENSCHRIFT,
SCHAPER, HANNOVER, DE, vol. 100, no. 5, 1 May
1993 (1993-05-01), pages 198-203, XP000610213
ISSN: 0012-0847**

- **KIENZLE E ET AL: "STRUVITE DIET IN CATS:
EFFECT OF AMMONIUM CHLORIDE AND
CARBONATES ON ACID BASE BALANCE OF
CATS1,2" JOURNAL OF NUTRITION, WISTAR
INSTITUTE OF ANATOMY AND BIOLOGY,
PHILADELPHIA, PA, US, vol. 124, no. 12S, 1
December 1994 (1994-12-01), pages 2652S-
2659S, XP000610052 ISSN: 0022-3166**

- **YAMKA, R.M., ET AL: "The Prediction of Urine pH
Using Dietary Cations and Anions in Cats Fed Dry
and Wet Foods" INTERN J APPL RES VET MED,
vol. 4, no. 1, 2006, pages 58-66, XP002523086**

- **ROCHE J.R. ET AL.: 'Dietary Cation-anion
Difference and Health and Production of Pasture-
fed Dairy Cows' JOURNAL OF DAIRY SCIENCE
vol. 86, no. 3, 2003, pages 979 - 987, XP003010134**

- **STEVENSON AE ET AL: "Comparison of urine
composition of healthy Labrador retrievers and
miniature schnauzers", AMERICAN JOURNAL
OF VETERINARY RESEARCH, AMERICAN
VETERINARY MEDICINE ASSOCIATION, US, vol.
62, no. 11, 1 January 2000 (2000-01-01), pages
1782-1786, XP009160033, ISSN: 0002-9645**

- **SMITH, BRIGITTE: "Effect of lifestage on urinary
tract health in the cat", WALTHAM FOCUS, vol. 8,
no. 2, 1998, pages 32-32,**

**(Cont. next page)**

EP 1 907 839 B1

• **HOUSTON D M ET AL: "Evaluation of the efficacy of a commercial diet in the dissolution of felinestruvite bladder uroliths", VETERINARY THERAPEUTICS, VETERINARY LEARNING SYSTEMS, TRENTON, NJ, US, vol. 5, no. 3, 1 January 2004 (2004-01-01) , pages 187-201, XP008085363, ISSN: 1528-3593**

## Description

### CROSS-REFERENCE TO RELATED APPLICATIONS

This application claims priority to U.S. Provisional Application Serial No. 60/698,311 filed July 12, 2005.

### BACKGROUND OF THE INVENTION

#### Field of the Invention

This invention relates generally to methods for predicting urine pH and particularly to methods for predicting urine pH based upon the composition of a food consumed by an animal.

#### Description of the Prior Art

Urolithiasis is the presence of stones and the process of forming stones in the urinary tract, i.e., the kidney, bladder, and/or urethra. Struvite uroliths are stones in the urinary tract comprising the mineral struvite or magnesium ammonium phosphate hexahydrate. Calcium oxalate uroliths are stones in the urinary tract composed of the mineral calcium oxalate. These uroliths or stones are also referred to as calculi.

The formation of stones in the urinary tract is a significant clinical problem for animals, including companion animals such as dogs and cats. Bacterial urinary tract infection is an important predisposing factor for struvite uroliths. The cause of calcium oxalate uroliths remains unknown. Animal urine pH has been shown to be an important determinant in the prevention and treatment of stone formation. A reduction in urine pH has been shown to reduce the incidence of struvite uroliths. However, a decrease in urine pH may increase the risk of calcium oxalate uroliths.

Diets that lower urine pH may be beneficial in preventing recurrence of struvite uroliths, e.g., a magnesium restricted diet. A diet low in protein, magnesium, and phosphorus and high in salt may be useful to dissolve struvite stones. Diets that increase urine pH may be beneficial in preventing recurrence of calcium oxalate uroliths, e.g., a diet containing potassium citrate or similar compounds.

Dietary ingredients that affect urine pH include sulfur containing amino acids and metabolizable cations and anions. Cations such as calcium, magnesium, sodium and potassium, anions such as sulfur, phosphorus and chloride; and sulfur-containing amino acids such as taurine, methionine, and cysteine have been shown to directly affect urine pH in many animals, including cats, swine, cattle, horses, and rats. While these dietary ingredients are known to affect urine pH, there are no known accurate methods for evaluating a particular food and determining how such food will affect urine pH and urolithiasis if it is consumed by an animal. There is, therefore, a need for new methods for predicting urine pH for an animal consuming a particular diet or food and for new methods for preventing or treating urolithiasis. Markwell et al (The Journal of Nutrition, vol. 128, 1998, pages 2753S to 2575S) discloses the effect of diet on lower urinary tract diseases in cats.

Kienzle et al (Dtsch. tierarzh. wschr., vol 100, 1993, pages 169 to 208) discloses the use of base excess of cat foods in a method of predicting urinary pH.

Kienzle et al (The journal of Nutrition, vol 124, 1994, pages 2652S to 2659S ) discloses the effect of ammonium chloride and carbonates on acid base balance of cats.

### SUMMARY OF THE INVENTION

It is, therefore, an object of the present invention to provide methods and devices for predicting urine pH.

These and other objects are achieved using novel methods for predicting urine pH by determining the amount of selected cations, anions, and sulfur-containing amino acids in a food for consumption by the animal and predicting urine pH using a formula that equates the amount of such cations, anions, and sulfur-containing amino acids to urine pH. Devices useful for predicting urine pH, kits comprising devices useful for predicting urine pH, and various means for communicating information about or instructions for using the present invention are also provided.

Other and further objects, features, and advantages of the present invention will be readily apparent to those skilled in the art.

### DETAILED DESCRIPTION OF THE INVENTION

#### Definitions

The term "animal" means a human or other animal susceptible to or suffering from urolithiasis, including avian, bovine, canine, equine, feline, hicrine, murine, ovine, and porcine animals. Preferably, the animal is a canine or feline.

The term "urolithiasis agent" means any drug, food, or other compound or composition useful for preventing or treating urolithiasis, including compositions that alter animal urine pH when consumed by the animal, drugs that lower calcium in the blood, antibiotics, and anti-bacterials.

The symbol "*" in the formulas herein means that the elements on each side of the "*" are multiplied, e.g., (1.2*sodium) means that the amount of sodium in the food is multiplied by 1.2.

The term "in conjunction" means that one or more of the foods, compositions, or compounds (e.g., urolithiasis agents) as provided by the description are administered to an animal (1) together in a food composition or (2) separately at the same or different frequency using the same or different administration routes at about the same time or periodically. "Periodically" means that the compositions, food compositions, and compounds are administered on a dosage schedule acceptable for a specific composition, food composition, and compound and that the food compositions are administered or fed to an animal routinely as appropriate for the particular animal. "About the same time" generally means that the compositions, composition components, urolithiasis agents, and food compositions are administered at the same time or within about 72 hours of each other. In conjunction specifically includes administration schemes wherein urolithiasis agents are administered for a prescribed period and the compositions are administered indefinitely.

The term "single package" means that the components of a kit are physically associated in or with one or more containers and considered a unit for manufacture, distribution, sale, or use. Containers include, but are not limited to, bags, boxes, bottles, shrink wrap packages, stapled or otherwise affixed components, or combinations thereof.

The term "virtual package" means that the components of a kit are associated by directions on one or more physical or virtual kit components instructing the user how to obtain the other components, e.g., in a bag containing one component and directions instructing the user to go to a website, contact a recorded message, view a visual message, or contact a caregiver or instructor to obtain instructions on how to use the kit.

This invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise, e.g., reference to "a method" or "a food composition" includes a plurality of such methods or compositions. Similarly, the words "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

Unless specified otherwise, the amounts of cations, anions, and sulfur-containing amino acids are measured in percent (%) on a dry matter (DM) basis.

The Invention

In one aspect, the present invention provides a method for predicting urine pH for an animal comprising determining the amount of selected cations, anions, and sulfur-containing amino acids in a food for consumption by the animal and predicting urine pH using a formula that equates the amount of such cations, anions, and sulfur-containing amino acids to urine pH, wherein the animal is a feline. The invention is based upon the novel discovery that the amounts of certain cations, anions, and sulfur-containing amino acids in a food can be used to predict urine pH and the discovery of the formula or algorithm that equates such amounts to urine pH. The invention is useful for reducing the number of in vitro and in vivo studies required to develop animal foods that will not adversely affect animal urine pH when consumed.

In one embodiment, the invention provides a method for predicting urine pH for an animal comprising determining the amount of the cations sodium, potassium, calcium, and magnesium; the anions chloride, sulfur, and phosphorus; and the sulfur-containing amino acids methionine and cystine in a food for consumption by the animal and predicting urine pH using the formula (Formula 1):

$$\text{Urine pH} = AA + (AB*\text{sodium}) + (AC*\text{potassium}) - (AD*\text{chloride}) - (AE*\text{sulfur}) + (AF*\text{calcium}) + (AG*\text{magnesium}) - (AH*\text{phosphorus}) - (AI*\text{methionine}) - (AJ*\text{cystine}),$$

where AA is from 5.5 to 7.5; AB is from 1.0 to 1.3; AC is from 0.6 to 0.9; AD is from 0.6 to 0.9; AE is from 0.4 to 0.9; AF is from 0.8 to 0.3; AG is from 1.0 to 1.5; AH is from 0.5 to 1.0; AI is from 0.1 to 0.5; and AJ is from 0.1 to 0.5, wherein the amounts of cations, anions and sulfur-containing amino acids are measured in percent on a dry matter basis and wherein the animal is a feline.

In one embodiment, the formula is urine pH = 7.05 + (1.15*sodium) + (0.72*potassium) - (0.75*chloride) - (0.46\*sulfur) + (0.12\*calcium) + (1.28\*magnesium) - (0.65\*phosphorus) - (0.22\*methionine) - (0.27\*cystine). In another, the formula is urine pH = 6.99 + (1.29\*sodium) + (0.78\*potassium) - (0.81\*chloride) - (0.49\*sulfur) + (0.12\*calcium) + (1.22\*magnesium) - (0.60\*phosphorus) - (0.22\*methionine) - (0.27\*cystine).

In another embodiment, the invention provides a method for predicting urine pH for an animal comprising determining the amount of the cations sodium, potassium, and magnesium; the anions chloride, sulfur, and phosphorus; and the sulfur-containing amino acids methionine and cystine in a wet food for consumption by the animal and predicting urine pH using the formula (Formula 2):

$$\text{Urine pH} = \text{WA} + (\text{WB*sodium}) + (\text{WC*potassium}) - (\text{WD*chloride}). - (\text{WE*sulfur}) + (\text{WG*magnesium}) - (\text{WH*phosphorus}) - (\text{WPmethionine}) - (\text{WJ*cystine}), \text{ where WA is from}$$

where WA is from 5.5 to 7.5; WB is from 1.2 to 1.5; WC is from 0.8 to 1.2; WD is from 1.0 to 1.3; WE is from 0.1 to 0.9; WG is from 3.5 to 5.5; WH is from 0.7 to 1.3; W1 is from 0.3 to 0.7; and WJ is from 0.3 to 0.8 wherein the amounts of cations, anions and sulfur-containing amino acids are measured in percent on a dry matter basis and wherein the animal is a feline.

In one embodiment, the formula is urine pH = 7.02 + (1.38\*sodium) + (0.99\*potassium) - (1.12\*chloride) - (0.29\*sulfur) + (4.51\*magnesium) - (0.99\*phosphorus) - (0.45\*methionine) + (0.50\*cystine). In another, the formula is urine pH = 7.03 + (1.43\*sodium) + (0.93\*potassium) - (1.16\*chloride) - (0.30\*sulfur) + (4.76\*magnesium) - (0.92\*phosphorus) - (0.41 Methionine) + (0.34\*cystine).

In a further embodiment, the invention provides a method for predicting urine pH for an animal comprising determining the amount of the cations sodium, potassium, calcium, and magnesium; and the anions chloride, sulfur, and phosphorus in a dry food for consumption by the animal and predicting urine pH using the formula (Formula 3):

$$\text{Urine pH} = \text{DA} + (\text{DB*sodium}) + (\text{DC*potassium}) - (\text{DD*chloride}) - (\text{DE*sulfur}) + (\text{DF*calcium}) + (\text{DG*magnesium}) - (\text{DH*phosphorus}),$$

where DA is from 5.5 to 7.5; DB is from 1.0 to 1.3; DC is from 0.6 to 0.9; DD is from 0.6 to 0.9; DE is from 0.4 to 0.6; DF is from 0.8 to 0.3; DG is from 1.0 to 1.5; and DH is from 0.5 to 1.0, wherein the amounts of cations, anions and sulfur-containing amino acids are measured in percent on a dry matter basis and wherein the animal is a feline.

In one embodiment, the formula is urine pH = 7.10 + (1.03\*sodium) + (0.98\*potassium) - (0.83\*chloride) - (1.70\*sulfur) + (0.85\*calcium) + (2.07\*magnesium) - (1.15\*phosphorus). In another, the formula is urine pH = 7.03 + (1.00\*sodium) + (1.00\*potassium) - (0.93\*chloride) - (1.61\*sulfur) + (0.89\*calcium) + (1.58\*magnesium) - (1.04\*phosphorus).

Methods for determining nutrient amounts, metabolite amounts, and urine pH are well known to skilled artisans.

In another aspect, the present invention provides a device suitable for predicting urine pH for an animal, comprising a calculator or computer, that, when given the amount of selected cations, anions and sulfur-containing amino acids in a food for consumption the animal, uses formula 1 of claim 1, or formula 2 of claim 2, or formula 3 of claim 3, that equates the amount of such cations, anions and sulfur-containing amino acids to a urine pH, in a method for predicting urine pH, wherein the animal is a feline.

The present invention further provides a device suitable for predicting urine pH for an animal comprising a website that incorporates or utilises a software program, or a software program, when run on a suitable computer-controlled apparatus, that, when given the amount of selected cations, anions and sulfur-containing amino acids in food for consumption by the animal, uses formula 1 of claim 1, or formula 2 of claim 2, or formula 3 of claim 3, that equates the amount of such cations, anions and sulfur-containing amino acids to urine pH, in a method for predicting urine pH, wherein the animal is feline.

In the device of the present invention, the calculator or computer or website or software program accepts input from a user, comprising the amount of sodium, potassium, calcium, magnesium, chloride, sulfur, phosphorus, methionine, in a food for consumption by an animal and utilizes all or a subset of the input, and one or more of Formula 1 of claim 1, Formula 2 of claim 2, and Formula 3 of claim 3 ,to predict urine pH in the animal that consumes the food.

The description further provides a method for preventing or treating urolithiasis. The method comprises determining a desirable urine pH range for an animal; choosing a food containing selected cations, anions, and sulfur-containing amino acids; predicting urine pH using a formula that equates the amount of such cations, anions, and sulfur-containing amino acids in the food to urine pH in the animal that consumes the food; and feeding an urolithiasis preventing or treating amount of the food to the animal if the predicted urine pH is in the desirable pH range.

In one embodiment, (1) the desirable pH range is from about 5.5 to 6.5, (2) the cations, anions, and sulfur-containing amino acids are all or a subset according to the methods of the present invention of the cations sodium, potassium, calcium, and magnesium; the anions chloride, sulfur, and phosphorus, and the sulfur-containing amino acids methionine and cystine, and (3) the formula is Formula 1, Formula 2, or Formula 3 as appropriate for the chosen food and the selected cations, anions, and sulfur-containing amino acids. In one embodiment, the method further comprises feeding the food in conjunction with one or more urolithiasis agents.

In a further aspect, the description provides a kit useful for predicting urine pH for an animal and/or for preventing or treating urolithiasis comprising in separate containers in a single package or in a virtual package, as appropriate for the kit component, a device of the present invention and one or more of (1) a food suitable for animal consumption, (2) an urolithiasis agent, (3) a urine pH diagnostic device, (4) a means for communicating information about or instructions for using urine pH diagnostic devices, (5) a means for communicating information about or instructions for adjusting or controlling urine pH, (6) a means for communicating information about or instructions for using the methods, devices, and kits of the present invention to predict urine pH, and (7) a means for communicating information about or instructions for preventing or treating urolithiasis. The kit components are typically in a separate package, in or on the package with one of the other kit components, or in a virtual package, as appropriate for the type of kit component. When the kit comprises a virtual package, the kit is limited to the instructions in a virtual environment in combination with one or more of the other physical kit components. In one embodiment, the food suitable for animal consumption comprises cations, anions, and sulfur-containing amino acids in amounts predicted by the present invention to cause urine pH to be in a desirable range.

In another aspect, the description provides a means for communicating information about or instructions for (1) using the methods, devices, and kits of the present invention to predict urine pH, (2) using the methods, devices, and kits of the present invention to prevent or treat urolithiasis, and (3) using urine pH diagnostic devices of the present invention. The communicating means comprises a document, digital storage media, optical storage media, audio presentation, or visual display containing the information or instructions. Preferably, the communication is a displayed website or a brochure, product label, package insert, advertisement, or visual display containing such information or instructions. Useful information and instructions include, but are not limited to, contact information for animals or their caregivers to use if they have a question about the invention and its use and how to use the present invention to calculate urine pH and manage urolithiasis. The communication means is useful for instructing an animal or its caregiver on the benefits of using the present invention.

The composition of foods suitable for consumption by an animal is known to skilled artisans. Typical food ingredients include but are not limited to fats, carbohydrates, proteins, fibers, and nutrients such as vitamins, minerals, and trace elements. Skilled artisans can select the amount and type of food ingredients for a typical food based upon the dietary requirements of the animal, e.g., the animal's species, age, size, weight, health, and function.

The fat and carbohydrate food ingredient is obtained from a variety of sources such as animal fat, fish oil, vegetable oil, meat, meat by-products, grains, other animal or plant sources, and mixtures thereof. Grains include wheat, corn, barley, grain sorghum, and rice.

The protein food ingredient is obtained from a variety sources such as plants, animals, or both. Animal protein includes meat, meat by-products, dairy, and eggs. Meats include the flesh from poultry, fish, and animals such as cattle, swine, sheep, goats, and the like. Meat by-products include lungs, kidneys, brain, livers, stomachs, and intestines. The protein food ingredient may also be free amino acids and/or peptides. Preferably, the protein food ingredient comprises meat, a meat byproduct, dairy products, or eggs.

The fiber food ingredient is obtained from a variety of sources such as vegetable fiber sources, e.g., cellulose, beet pulp, peanut hulls, and soy fiber.

The nutrients are obtained from a variety of sources known to skilled artisans, e.g., vitamin and mineral supplements and food ingredients. Vitamins and minerals can be included in amounts required to avoid deficiency and maintain health. These amounts are readily available in the art. The National Research Council (NRC) provides recommended amounts of such nutrients for farm animals. See, e.g., Nutrient Requirements of Swine (10th Rev. Ed., Nat'l Academy Press, Wash. D.C., 1998), Nutrient Requirements of Poultry (9th Rev. Ed., Nat'l Academy Press, Wash. D.C., 1994), Nutrient Requirements of Horses (5th Rev. Ed., Nat'l Academy Press, Wash. D.C., 1989). The American Feed Control Officials (AAFCO) provides recommended amounts of such nutrients for dogs and cats. See American Feed Control Officials, Inc., Official publication, pp. 129-137 (2004). Vitamins generally useful as food additives include vitamin A, vitamin B1, vitamin B2, vitamin B6, vitamin B 12, vitamin D, biotin, vitamin K, folic acid, inositol, niacin, and pantothenic acid. Minerals and trace elements useful as food additives include calcium, phosphorus, sodium, potassium, magnesium, copper, zinc, chloride, iron, selenium, iodine, and iron.

The food compositions may contain additional ingredients such as vitamins, minerals, fillers, palatability enhancers, binding agents, flavors, stabilizers, emulsifiers, sweeteners, colorants, buffers, salts, coatings, and the like known to skilled artisans. Stabilizers include substances that tend to increase the shelf life of the composition such as preservatives, synergists and sequestrants, packaging gases, stabilizers, emulsifiers, thickeners, gelling agents, and humectants. Examples of emulsifiers and/or thickening agents include gelatin, cellulose ethers, starch, starch esters, starch ethers, and modified starches. Specific amounts for each composition component, food ingredient, and other ingredients will depend on a variety of factors such as the particular components and ingredients included in the composition; the species of animal; the animal's age, body weight, general health, sex, and diet; the animal's consumption rate; the type of urolithiasis being treated; and the like. Therefore, the component and ingredient amounts may vary widely and may deviate from the preferred proportions described herein.

The food compositions may be canned or wet foods known to skilled artisans. Typically, ground animal proteinaceous tissues are mixed with the other ingredients such as fish oils, cereal grains, balancing ingredients, special purpose additives (e.g., vitamin and mineral mixtures, inorganic salts, cellulose and beet pulp, bulking agents, and the like) and water in amounts sufficient for processing. These ingredients are mixed in a vessel suitable for heating while blending the components. Heating of the mixture is effected using any suitable manner, e.g., direct steam injection or using a vessel fitted with a heat exchanger. Following the addition of the last ingredient, the mixture is heated to a temperature of from about 10°C (50 °F) to about 100°C (212 °F). Temperatures outside this range are acceptable but may be commercially impractical without use of other processing aids. When heated to the appropriate temperature, the material will typically be in the form of a thick liquid. The thick liquid is filled into cans. A lid is applied, and the container is hermetically sealed. The sealed can is then placed into conventional equipment designed to sterilize the contents. Sterilization is usually accomplished by heating to temperatures of greater than about 110°C (230°F) for an appropriate time depending on the temperature used, the composition, and similar factors. The compositions of the present invention can be added to the food compositions before, during, or after preparation.

The food compositions may be dry foods known to skilled artisans. Typically, dry ingredients such as animal protein, plant protein, grains, and the like are ground and mixed together. Moist or liquid ingredients, including fats, oils, animal protein, water, and the like are then added to and mixed with the dry mix. The mixture is then processed into dry food pieces.

The food compositions may be can be in any form useful for feeding the composition to an animal, e.g., kibbles, treats, and toys for animal food. Kibbles are generally formed using an extrusion process in which the mixture of dry and wet ingredients is subjected to mechanical work at a high pressure and temperature and forced through small openings and cut off into kibble by a rotating knife. The wet kibble is then dried and optionally coated with one or more topical coatings such as flavors, fats, oils, powders, and the like. Kibble also can be made from the dough using a baking process, rather than extrusion, wherein the dough is placed into a mold before dry-heat processing. Treats include compositions that are given to an animal to entice the animal to eat during a non-meal time, e.g., dog bones or biscuits for canines. Treats may be nutritional wherein the composition comprises one or more nutrients or and may have a food-like composition. Non-nutritional treats encompass any other treats that are non-toxic. The composition or components are coated onto the treat, incorporated into the treat, or both. Treats of the present description can be prepared by an extrusion or baking process similar to those used for dry food. Other processes also may be used to either coat the composition on the exterior of existing treat forms or inject the composition into an existing treat form. Toys include chewable toys such as artificial bones and food compositions shaped to resemble natural foods that are appealing to the animal. The food composition of the present description can comprise the toy or can form a coating on the surface of the toy or on the surface of a component of the toy. The composition can be incorporated partially or fully throughout the toy or both. In one embodiment, the composition is orally accessible by the intended user. There are a wide range of suitable toys known to skilled artisans, e.g., as shown in US Patent Nos. 5,339,771 and 5,419,283. The present description provides partially consumable toys, e.g., toys comprising plastic components, and fully consumable toys, e.g., various artificial bones and similar foods. Further, the description provides toys for both human and non-human use, particularly toys for companion, farm, and zoo animal use, and more particularly for feline and canine use.

EXAMPLES

This invention can be further illustrated by the following examples of preferred embodiments, although it will be understood that these examples are included merely for purposes of illustration and are not intended to limit the scope of the invention unless otherwise specifically indicated.

Example 1

132 foods (82 dry foods and 50 wet foods) were fed to groups of ten adult (mean = 8.5 years of age) cats (felines) to determine the urine pH of cats fed each food. The food was fed for a period of seven days and on days 5 to 7 of the test, urine pH was determined from urine collected at 0730 and 1430 hours.

The cats were cared for in accordance with Institutional Animal Care and Use Committee protocols. The cat's primary living space was cleaned twice daily. Throughout the duration of the experiment, cats were exercised daily and human interaction included but was not limited to play (toys), grooming and other human-cat interactions (i.e. petting). Water was available ad libitum throughout the entire experiment.

The range of nutrients for all dry (82) and wet (50) foods are shown in Tables 1 and 2. Each food was formulated in accordance with the Association of American Feed Control Officials 11 nutrient guide for cats and balanced to meet growth and adult maintenance. Each food was fed to a group of ten cats to maintain body weight. Each day food was offered at 6 hour intervals at 0600, 1200, 1800 and 2400 by an automatic feeding system a to ensure that fresh food was available at all times. Excess food was removed daily and orts were weighed and recorded. Food samples were collected for nutrient content analysis.

Table 1

| Analyzed Nutrients and Observed Urine pH of the 82 Dry Foods Used in Feline Urine pH Studies | | | | |
|---|---|---|---|---|
| Nutrient, 100% DM Basis | Minimum | Maximum | Average | Standard Deviation |
| Sodium | 0.198 | 0.647 | 0.357 | 0.087 |
| Potassium | 0.307 | 1.542 | 0.805 | 0.207 |
| Chloride | 0.476 | 1.321 | 0.841 | 0.173 |
| Sulfur | 0.465 | 1.149 | 0.722 | 0.172 |
| Calcium | 0.585 | 1.269 | 0.897 | 0.148 |
| Magnesium | 0.043 | 0.182 | 0.088 | 0.039 |
| Phosphorus | 0.486 | 1.064 | 0.755 | 0.095 |
| Methionine | 0.493 | 3.546 | 0.931 | 0.401 |
| Cystine | 0.378 | 1.070 | 0.663 | 0.164 |
| Observed Urine pH | 5.79 | 7.12 | 6.40 | 0.32 |

Table 2

| Analyzed Nutrients and Observed Urine pH of the 50 Wet Foods Used in Feline Urine pH Studies | | | | |
|---|---|---|---|---|
| Nutrient, 100% DM Basis | Minimum | Maximum | Average | Standard Deviation |
| Sodium | 0.267 | 1.227 | 0.394 | 0.170 |
| Potassium | 0.643 | 1.074 | 0.900 | 0.102 |
| Chloride | 0.172 | 2.876 | 0.883 | 0.416 |
| Sulfur | 0.237 | 2.763 | 0.851 | 0.323 |
| Calcium | 0.469 | 1.338 | 0.945 | 0.214 |
| Magnesium | 0.055 | 0.351 | 0.088 | 0.045 |
| Phosphorus | 0.524 | 1.104 | 0.811 | 0.123 |
| Methionine | 0.345 | 2.005 | 1.353 | 0.338 |
| Cystine | 0.251 | 0.866 | 0.408 | 0.112 |
| Observed Urine pH | 5.85 | 6.98 | 6.40 | 0.288 |

Urine samples were collected twice per day during the course of the one week study at 0730 and 1430 on days 5, 6, and 7. Cat litter boxes were removed from cages at 0600 and returned following the 0730 collection, removed at 1100, and returned following the 1430 collection on days 5, 6, and 7. Urine samples were collected from each cat into a numbered cup by manually expressing the cat's bladder. This ensured that the urine pH was consistent with the actual urine pH in the cat's bladder and prevented contamination from feces if collected naturally. Urine pH was measured using a pH meter.

All feed samples were analyzed for moisture (930.15), calcium (968.08), sodium (968.08), potassium (968.08), magnesium (968.08), chloride (969.10), sulfur (923.01), phosphorus (965.17), methionine and cystine (994.12) according to the Association of Official Analytical Chemists.

After the conclusion of all 132 urine pH studies, the nutrients and observed urine pH values were used to challenge previously published models. These data were then plotted. However, these models failed to accurately predict the average urine pH of the foods used in the current study ($r2 = 0.23$, 0.23 and 0.10, respectively).

A new model was developed using previously known cations and anions that effect urine pH with the addition of sulfur in cats fed dry and can foods. Individual mean urine pH per cat (1320 individual urine pH means) was then regressed to the nutrient content (100% dry matter basis) of the food consumed.

Stepwise regression was used to determine which cations and anions were of predictive importance. The cations included in the dry and wet model were sodium, calcium, potassium and magnesium, whereas the anions were chloride, sulfur, phosphorus and the amino acids methionine and cystine. The results are shown in Table 3. The analysis resulted in a prediction equation for foods. The new model accounted for 34% variation in individual (n=1320) observed urine pH and 51% of the variation observed in average (n=132) urine pH in the cats fed 132 foods.

## Table 3

### Urine pH Prediction Models Determined by Stepwise Regression for Individual Cats (n=1320) Fed 132 Dry and Wet Foods Using the Nutrient Components of the Food (% Dry Matter Basis)

| Model | Sodium | Potassium | Chloride | Sulfur | Calcium |
|---|---|---|---|---|---|
| 1 | -- | -- | -0.29 ± 0.04 | -- | -- |
| 2 | -- | -- | -0.49 ± 0.04 | -- | -- |
| 3 | -- | -- | -0.55 ± 0.04 | -0.46 ± 0.04 | -- |
| 4 | -- | -- | -0.56 ± 0.04 | -0.49 ± 0.04 | -- |
| 5 | -- | -- | -0.56 ± 0.04 | -0.48 ± 0.04 | -- |
| 6 | -- | -- | -0.53 ± 0.04 | -0.40 ± 0.04 | -- |
| 7 | -- | 0.42 ± 0.07 | -0.47 ± 0.04 | -0.45 ± 0.04 | -- |
| 8 | 1.12 ± 0.14 | 0.72 ± 0.08 | -0.75 ± 0.05 | -0.44 ± 0.04 | -- |
| 9 | 1.15 ± 0.14 | 0.72 ± 0.08 | -0.75 ± 0.05 | -0.46 ± 0.04 | 0.17 ± 0.07 |

## Table 3, Continued

| Model | Magnesium | Phosphorus | Methionine | Cystine | Intercept | R2 |
|---|---|---|---|---|---|---|
| 1 | -- | -- | -- | -- | 6.65 ± 0.03 | 0.05 |
| 2 | 3.23 ± 0.26 | -- | -- | -- | 6.54 ± 0.03 | 0.15 |
| 3 | 3.93 ± 0.26 | -- | -- | -- | 6.88 ± 0.04 | 0.23 |
| 4 | 3.95 ± 0.25 | -- | -- | -0.30 ± 0.05 | 7.08 ± 0.05 | 0.25 |
| 5 | 4.06 ± 0.25 | -0.50 ± 0.08 | -- | -0.36 ± 0.05 | 7.49 ± 0.09 | 0.27 |
| 6 | 3.86 ± 0.25 | -0.47 ± 0.08 | -0.13 ± 0.02 | -0.45 ± 0.05 | 7.59 ± 0.09 | 0.29 |
| 7 | 2.77 ± 0.31 | -0.48 ± 0.08 | -0.19 ± 0.03 | -0.43 ± 0.05 | 7.36 ± 0.09 | 0.31 |
| 8 | 1.33 ± 0.35 | -0.53 ± 0.08 | -0.23 ± 0.03 | -0.29 ± 0.05 | 7.09 ± 0.10 | 0.33 |
| 9 | 1.28 ± 0.35 | -0.65 ± 0.11 | -0.22 ± 0.03 | -0.27 ± 0.05 | 7.05 ± 0.10 | 0.34 |

Example 2

To determine if separating the food types (dry vs. wet) resulted in higher accuracy in urine pH prediction two more models (dry only and wet only) were developed. The cations included in the wet model were sodium, potassium and magnesium, whereas the anions were chloride, sulfur, phosphorus and the sulfur amino acids methionine and cystine. Calcium was excluded from this model. The results are shown in Table 4. The analysis resulted in a prediction equation for wet foods. The new model accounted for 34% variation in individual (n=1320) observed urine pH and 51 % of the variation observed in average (n=132) urine pH in the cats fed 132 foods. The new model accounted for 39% variation in individual (n=500) observed urine pH and 60% of the variation observed in average (n=50) urine pH in the cats fed 50 wet foods.

Table 4

Urine pH Prediction Models Determined by Stepwise Regression for Individual Cats (n=500) Fed 50 Wet Foods Using the Nutrient Components of the Food (% Dry Matter Basis)

| Model | Sodium | Potassium | Chloride | Sulfur | Calcium |
|---|---|---|---|---|---|
| 1 | -- | -- | -- | -- | -- |
| 2 | -- | -- | -- | -0.25 ± 0.05 | -- |
| 3 | -- | -- | -- | -0.27 ± 0.05 | -- |
| 4 | -- | -- | -0.17 ± 0.04 | -0.31 ± 0.05 | -- |
| 5 | -- | -- | -0.76 ± 0.08 | -0.29 ± 0.05 | -- |
| 6 | 1.30 ± 0.20 | -- | -1.09 ± 0.09 | -0.22 ± 0.05 | -- |
| 7 | 1.48 ± 0.20 | 0.80 ± 0.15 | -1.15 ± 0.09 | -0.24 ± 0.04 | -- |
| 8 | 1.38 ± 0.20 | 0.99 ± 0.16 | -1.12 ± 0.09 | -0.29 ± 0.05 | -- |

Table 4, Continued

| Model | Magnesium | Phosphorus | Methionine | Cystine | Intercept | R2 |
|---|---|---|---|---|---|---|
| 1 | -- | -- | -0.32 ± 0.05 | -- | 6.84 ± 0.07 | 0.08 |
| 2 | -- | -- | -0.30 ± 0.05 | -- | 7.02 ± 0.07 | 0.13 |
| 3 | -- | -0.47 ± 0.13 | -0.35 ± 0.05 | -- | 7.50 ± 0.15 | 0.15 |
| 4 | -- | -0.63 ± 0.14 | -0.38 ± 0.05 | -- | 7.85 ± 0.17 | 0.18 |
| 5 | 6.10 ± 0.73 | -1.03 ± 0.14 | -0.29 ± 0.05 | -- | 8.01 ± 0.16 | 0.28 |
| 6 | 4.85 ± 0.73 | -0.84 ± 0.14 | -0.24 ± 0.05 | -- | 7.62 ± 0.17 | 0.34 |
| 7 | 4.59 ± 0.71 | -0.89 ± 0.13 | -0.33 ± 0.05 | -- | 7.10 ± 0.19 | 0.37 |
| 8 | 4.51 ± 0.70 | -0.99 ± 0.13 | -0.45 ± 0.06 | 0.50 ± 0.16 | 7.02 ± 0.19 | 0.39 |

Example 3

The cations included in the dry model were sodium, potassium, magnesium and calcium, whereas the anions were chloride, sulfur and phosphorus. Methionine and cystine were excluded from this model. The results are shown in Table 5. The analysis resulted in a prediction equation for dry foods. The new model accounted for 51 % variation in individual (n=820) observed urine pH and 74% of the variation observed in average (n=82) urine pH in the cats fed 82 dry foods.

Table 5

Urine pH Prediction Models Determined by Stepwise Regression for Individual Cats (n=820) Fed 82 Dry Foods Using the Nutrient Components of the Food (% Dry Matter Basis).

| Model | Sodium | Potassium | Chloride | Sulfur | Calcium |
|---|---|---|---|---|---|
| 1 | -- | -- | -1.10 ± 0.07 | -- | -- |
| 2 | -- | 0.46 ± 0.06 | -1.11 ± 0.07 | -- | -- |
| 3 | -- | 0.94 ± 0.06 | -0.90 ± 0.06 | -0.98 ± 0.08 | -- |
| 4 | 1.21 ± 0.14 | 1.17 ± 0.07 | -1.12 ± 0.07 | -1.28 ± 0.08 | -- |
| 5 | 0.98 ± 0.15 | 1.13 ± 0.07 | -1.14 ± 0.06 | -1.35 ± 0.08 | 0.39 ± 0.08 |
| 6 | 1.20 ± 0.15 | 1.19 ± 0.07 | -1.03 ± 0.06 | -1.51 ± 0.08 | 0.85 ± 0.10 |
| 7 | 1.03 ± 0.16 | 0.98 ± 0.09 | -0.83 ± 0.08 | -1.70 ± 0.10 | 0.85 ± 0.10 |

Table 5, Continued

| Model | Magnesium | Phosphorus | Methionine | Cystine | Intercept | R2 |
|---|---|---|---|---|---|---|
| 1 | -- | -- | -- | -- | 7.32 ± 0.06 | 0.24 |
| 2 | -- | -- | -- | -- | 6.96 ± 0.07 | 0.30 |
| 3 | -- | -- | -- | -- | 7.11 ± 0.07 | 0.41 |
| 4 | -- | -- | -- | -- | 6.89 ± 0.07 | 0.46 |
| 5 | -- | -- | -- | -- | 6.72 ± 0.08 | 0.47 |
| 6 | -- | -1.09 ± 0.16 | -- | -- | 7.01 ± 0.09 | 0.50 |
| 7 | 2.07 ± 0.57 | -1.15 ± 0.16 | -- | -- | 7.10 ± 0.09 | 0.51 |

Results

The results from the stepwise regression analysis show which cations, anions and sulfur containing amino acids were of importance for urine pH prediction. Three models were developed for urine pH prediction. These models included foods, wet foods, and dry foods. The cations included in all models were sodium, potassium and magnesium. Calcium was included in the wet and dry and dry food model only. The anions for all models were chloride, sulfur and phosphorus. Including sulfur in the model allowed for the exclusion of methionine and cysteine from the dry model. The results show that urine pH can be predicted from the nutrient components of the food thus reducing the number of animal studies in order to optimize urine pH (for struvite and/or oxalate prevention) for specific products. Separate formulas can be used for dry and wet foods to maintain accuracy.

In the specification, there have been disclosed typical preferred embodiments of the invention. They are for illustrative purposes only and are not intended to limit scope of the claims.

**Claims**

1. A method of predicting urine pH for an animal comprising:

determining the amount of selected cations sodium, potassium, calcium and magnesium, and anions chloride, sulfur and phosphorus, and sulfur-containing amino acids methionine and cystine in a food for consumption by the animal; and
predicting urine pH using the formula (Formula 1):

$$\text{Urine pH} = \text{AA} + (\text{AB*sodium}) + (\text{AC*potassium}) - (\text{AD*chloride}) - (\text{AE*sulfur}) + (\text{AF*calcium}) + (\text{AG*magnesium}) - (\text{AH*phosphorus}) - (\text{AI*methionine}) - (\text{AJ*cystine})$$

Where AA is from 5.5 to 7.5; AB is from 1.0 to 1.3; AC is from 0.6 to 0.9; AD is from 0.6 to 0.9; AE is from 0.4 to 0.9; AF is from 0.8 to 0.3, AG is from 1 to 1.5; AH is from 0.5 to 1.0; AI is from 0.1 to 0.5; and AJ is from 0.1 to 0.5, wherein the amounts of cations, anions and sulfur-containing amino acids are measured in percent on a dry matter basis
and wherein the animal is a feline.

2. A method of predicting urine pH for an animal comprising:

determining the amount of selected cations sodium, potassium, and magnesium, the anions are chloride, sulfur, and phosphorus; and the sulfur-containing amino acids methionine and cystine in a food for consumption by the animal;
wherein the food is a wet food,
and
predicting urine pH using the formula (Formula 2):

$$\text{Urine pH} = \text{WA} + (\text{WB*sodium}) + (\text{WC*potassium}) - (\text{WD*chloride}) - (\text{WE*sulfur}) + (\text{WG*magnesium}) - (\text{WH*phosphorus}) - (\text{WI*methionine}) - (\text{WJ*cystine}); \text{ where WA is}$$

where WA is from 5.5 to 7.5; WB is from 1.2 to 1.5; WC is from 0.8 to 1.2; WD is from 1.0 to 1.3; WE is from 0.1 to 0.9; WG is from 3.5 to 5.5; WH is from 0.7 to 1.3; WI is from 0.3 to 0.7; and WJ is from 0.3 to 0.8, wherein the amounts of cations, anions and sulfur-containing amino acids are measured in percent in a dry matter basis
and wherein the animal is a feline.

3. A method of predicting urine pH for an animal comprising:

determining the amount of selected cations sodium, potassium, calcium, and magnesium, and the anions chloride, sulfur, and phosphorus; in a food for consumption by the animal;
wherein the food is a dry food,
and
predicting urine pH using the formula (Formula 3):

$$\text{Urine pH} = \text{DA} + (\text{DB*sodium}) + (\text{DC*potassium}) - (\text{DD*chloride}) - (\text{DE*sulfur}) + (\text{DF*calcium}) + (\text{DG*magnesium}) - (\text{DH*phosphorus});$$

where DA is from 5.5 to 7.5; DB is from 1.0 to 1.3; DC is from 0.6 to 0.9; DD is from 0.6 to 0.9; DE is from 0.4 to 0.6; DF is from 0.8 to 0.3; DG is from 1.0 to 1.5; and DH is from 0.5 to 1.0, wherein the amounts of cations, anions and sulfur-containing amino acids are measured in percent in a dry matter basis
and wherein the animal is a feline.

4. A device suitable for predicting urine pH for an animal, comprising a calculator or computer, that, when given the amount of selected cations, anions and sulfur-containing amino acids in a food for consumption the animal, uses formula 1 of claim 1, or formula 2 of claim 2, or formula 3 of claim 3, that equates the amount of such cations, anions and sulfur-containing amino acids to a urine pH, in a method for predicting urine pH, wherein the animal is a feline.

5. A device suitable for predicting urine pH for an animal comprising a website that incorporates or utilises a software program, or a software program, when run on a suitable computer-controlled apparatus, that, when given the amount of selected cations, anions and sulfur-containing amino acids in food for consumption by the animal, uses formula 1 of claim 1, or formula 2 of claim 2, or formula 3 of claim 3, that equates the amount of such cations, anions and sulfur-containing amino acids to urine pH, in a method for predicting urine pH, wherein the animal is feline.

6. The device of claim 4 or claim 5, wherein the calculator or computer or website or software program accepts input from a user, comprising the amount of sodium, potassium, calcium, magnesium, chloride, sulfur, phosphorus, methionine, cystine in a food for consumption by an animal and utilizes all or a subset of the input, and one or more of Formula 1 of claim 1, Formula 2 of claim 2, and Formula 3 of claim 3 ,to predict urine pH in the animal that consumes the food.

**Patentansprüche**

1. Verfahren zur Vorausberechnung des pH-Werts von Urin bei einem Tier, bei dem:

   die Menge von ausgewählten Kationen Natrium, Kalium, Calcium und Magnesium und Anionen Chlorid, Schwefel und Phosphor, und schwefelhaltigen Aminosäuren Methionin und Cystin in einem Futter zum Verzehr durch das Tier bestimmt werden und
   der pH-Wert des Urins unter Verwendung der Formel (Formel 1) vorausberechnet wird:

   $$\text{pH-Wert von Urin} = AA + (AB*\text{Natrium}) + (AC*\text{Kalium}) - (AD*\text{Chlorid}) - (AE*\text{Schwefel}) + (AF*\text{Calcium}) + (AG*\text{Magnesium}) - (AH*\text{Phosphor}) - (AI*\text{Methionin}) - (AJ*\text{Cystin}),$$

   wobei AA 5,5 bis 7,5 beträgt, AB 1,0 bis 1,3 beträgt, AC 0,6 bis 0,9 beträgt, AD 0,6 bis 0,9 beträgt, AE 0,4 bis 0,9 beträgt, AF 0,8 bis 0,3 beträgt, AG 1 bis 1,5 beträgt, AH 0,5 bis 1,0 beträgt, AI 0,1 bis 0,5 beträgt und AJ 0,1 bis 0,5 beträgt,
   wobei die Mengen an Kationen, Anionen und schwefelhaltigen Aminosäuren in Prozent auf Basis der Trockenmasse gemessen werden,
   und wobei das Tier eine Katze ist.

2. Verfahren zur Vorausberechnung des pH-Werts von Urin bei einem Tier, bei dem:

   die Menge von ausgewählten Kationen Natrium, Kalium und Magnesium, die Anionen Chlorid, Schwefel und Phosphor sind, und schwefelhaltigen Aminosäuren Methionin und Cystin in einem Futter zum Verzehr durch das Tier bestimmt werden,
   wobei das Futter ein Feuchtfutter ist,
   und
   der pH-Wert von Urin unter Verwendung der Formel (Formel 2) vorausberechnet wird:

   $$\text{pH-Wert von Urin} = WA + (WB*\text{Natrium}) + (WC*\text{Kalium}) - (WD*\text{Chlorid}) - (WE*\text{Schwefel}) + (WG*\text{Magnesium}) - (WH*\text{Phosphor}) - (WI*\text{Methionin}) - (WJ*\text{Cystin}),$$

   wobei WA 5,5 bis 7,5 beträgt, WB 1,2 bis 1,5 beträgt, WC 0,8 bis 1,2 beträgt, WD 1,0 bis 1,3 beträgt, WE 0,1 bis 0,9 beträgt, WG 3,5 bis 5,5 beträgt, WH 0,7 bis 1,3 beträgt, WI 0,3 bis 0,7 beträgt und WJ 0,3 bis 0,8 beträgt,
   wobei die Mengen an Kationen, Anionen und schwefelhaltigen Aminosäuren in Prozent auf Basis der Trockenmasse gemessen werden,
   und wobei das Tier eine Katze ist.

3. Verfahren zur Vorausberechnung des pH-Werts von Urin bei einem Tier, bei dem:

   die Menge von ausgewählten Kationen Natrium, Kalium, Calcium und Magnesium und der Anionen Chlorid, Schwefel und Phosphor in einem Futter zum Verzehr durch das Tier bestimmt werden,
   wobei das Futter ein Trockenfutter ist,
   und

der pH-Wert von Urin unter Verwendung der Formel (Formel 3) vorausberechnet wird:

```
pH-Wert  von  Urin  =  DA  +  (DB*Natrium)  +  (DC*Kalium)  −
(DD*Chlorid) − (DE*Schwefel) + (DF*Calcium) + (DG*Magnesium)
− (DH*Phosphor),
```

wobei DA 5,5 bis 7,5 beträgt, DB 1,0 bis 1,3 beträgt, DC 0,6 bis 0,9 beträgt, DD 0,6 bis 0,9 beträgt, DE 0,4 bis 0,6 beträgt, DF 0,8 bis 0,3 beträgt, DG 1,0 bis 1,5 beträgt und DH 0,5 bis 1,0, beträgt,
wobei die Mengen an Kationen, Anionen und schwefelhaltigen Aminosäuren in Prozent auf Basis der Trockenmasse gemessen werden,
und wobei das Tier eine Katze ist.

4. Vorrichtung, die zur Vorausberechnung des pH-Werts von Urin bei einem Tier geeignet ist und die einen Rechner oder Computer umfasst, der bei Eingabe der Menge an ausgewählten Kationen, Anionen und schwefelhaltigen Aminosäuren in einem Futter zum Verzehr durch das Tier die Formel 1 gemäß Anspruch 1 oder Formel 2 gemäß Anspruch 2 oder Formel 3 gemäß Anspruch 3, die die Menge solcher Kationen, Anionen und schwefelhaltiger Aminosäuren in eine Beziehung zu dem pH-Wert von Urin setzt, in einem Verfahren zur Vorausberechnung des pH-Werts von Urin verwendet, wobei das Tier eine Katze ist.

5. Vorrichtung, die zur Vorausberechnung des pH-Werts von Urin bei einem Tier geeignet ist und die eine Website, die ein Software-Programm aufweist oder einsetzt, oder ein Software-Programm aufweist, wenn es auf einem geeigneten computergesteuerten Apparat ausgeführt wird, die bzw. das bei Eingabe der Menge an ausgewählten Kationen, Anionen und schwefelhaltigen Aminosäuren in einem Futter zum Verzehr durch das Tier die Formel 1 gemäß Anspruch 1 oder Formel 2 gemäß Anspruch 2 oder Formel 3 gemäß Anspruch 3, die die Menge solcher Kationen, Anionen und schwefelhaltiger Aminosäuren in eine Beziehung zu dem pH-Wert von Urin setzt, in einem Verfahren zur Vorausberechnung des pH-Werts von Urin verwendet, wobei das Tier eine Katze ist.

6. Vorrichtung nach Anspruch 4 oder Anspruch 5, wobei der Rechner oder Computer oder die Website oder das Software-Programm eine Eingabe von einem Anwender akzeptiert, einschließlich der Menge an Natrium, Kalium, Calcium, Magnesium, Chlor, Schwefel, Phosphor, Methionin, Cystin in einem Futter zum Verzehr durch ein Tier, und die die gesamte oder eine Teilmenge der Eingabe sowie eine oder mehrere von Formel 1 gemäß Anspruch 1, Formel 2 gemäß Anspruch 2 und Formel 3 gemäß Anspruch 3 einsetzt, um den pH-Wert von Urin bei dem Tier vorauszuberechnen, welches das Futter verzehrt.

## Revendications

1. Procédé de prévision du pH de l'urine d'un animal, comprenant :

la détermination de la quantité de cations de sodium, de potassium, de calcium et de magnésium, et d'anions de chlorure, de soufre et de phosphore, et d'acides aminés contenant du soufre, de la méthionine et de la cystine choisis dans un aliment destinée à la consommation animale ; et
la prévision du pH de l'urine en utilisant la formule (formule 1) :

$$pH\ de\ l'urine = AA + (AB * sodium) + (AC * potassium) - (AD * chlorure) - (AE * soufre) + (AF * calcium) + (AG * magnésium) - (AH * phosphore) - (AI * méthionine) - (AJ * cystine)$$

où AA va de 5,5 à 7,5 ; AB va de 1,0 à 1,3 ; AC va de 0,6 à 0,9 ; AD va de 0,6 à 0,9 ; AE va de 0,4 à 0,9 ; AF va de 0,8 à 0,3 ; AG va de 1 à 1,5 ; AH va de 0,5 à 1,0 ; AI va de 0,1 à 0,5 ; et AJ va de 0,1 à 0,5 ;
dans lequel les quantités de cations, d'anions et d'acides aminés contenant du soufre sont mesurées en pourcentage sur une base de matière sèche ; et
dans lequel l'animal est un félin.

**2.** Procédé de prévision du pH de l'urine d'un animal, comprenant :

la détermination de la quantité de cations de sodium, de potassium et de magnésium, et d'anions de chlorure, de soufre, et de phosphore, et d'acides aminés contenant du soufre, de la méthionine et de la cystine choisis dans un aliment destinée à la consommation animale ;
dans lequel l'aliment est un aliment humide ; et
la prévision du pH de l'urine en utilisant la formule (formule 2) :

$$\text{pH de l'urine} = WA + (WB * \text{sodium}) + (WC * \text{potassium}) - (WD * \text{chlorure}) -$$
$$(WE * \text{soufre}) + (WG * \text{magnésium}) - (WH * \text{phosphore}) - (WI * \text{méthionine}) -$$
$$(WJ * \text{cystine})$$

où WA va de 5,5 à 7,5 ; WB va de 1,2 à 1,5 ; WC va de 0,8 à 1,2 ; WD va de 1,0 à 1,3 ; WE va de 0,1 à 0,9 ; WG va de 3,5 à 5,5 ; WH va de 0,7 à 1,3 ; WI va de 0,3 à 0,7 ; et WJ va de 0,3 à 0,8 ;
dans lequel les quantités de cations, d'anions et d'acides aminés contenant du soufre sont mesurées en pourcentage sur une base de matière sèche
et dans lequel l'animal est un félin.

**3.** Procédé de prévision du pH de l'urine d'un animal, comprenant :

la détermination de la quantité de cations de sodium, de potassium, de calcium et de magnésium, et d'anions de chlorure, de soufre, et de phosphore, choisis dans un aliment destinée à la consommation animale ;
dans lequel l'aliment est un aliment sec ; et
la prévision du pH de l'urine en utilisant la formule (formule 3) :

$$\text{pH de l'urine} = DA + (DB * \text{sodium}) + (DC * \text{potassium}) - (DD * \text{chlorure}) -$$
$$(DE * \text{soufre}) + (DF * \text{calcium}) + (DG * \text{magnésium}) - (DH * \text{phosphore})$$

où DA va de 5,5 à 7,5 ; DB va de 1,0 à 1,3 ; DC va de 0,6 à 0,9 ; DD va de 0,6 à 0,9 ; DE va de 0,4 à 0,6 ; DF va de 0,8 à 0,3 ; DG va de 1,0 à 1,5 ; et DH va de 0,5 à 1,0 ;
dans lequel les quantités de cations, d'anions et d'acides aminés contenant du soufre sont mesurées en pourcentage sur une base de matière sèche
et dans lequel l'animal est un félin.

**4.** Dispositif approprié pour la prévision du pH de l'urine d'un animal, comprenant un calculateur ou un ordinateur, qui utilise, lorsque l'on a saisi la quantité de cations, d'anions et d'acides aminés contenant du soufre choisis dans un aliment destinée à la consommation animale, la formule 1 de la revendication 1, ou la formule 2 de la revendication 2, ou la formule 3 de la revendication 3, qui équivaut à la quantité de tels cations, anions et acides aminés contenant du soufre par rapport au pH de l'urine, suivant un procédé de prévision du pH de l'urine, dans lequel l'animal est un félin.

**5.** Dispositif approprié pour la prévision du pH de l'urine d'un animal, comprenant un site Internet qui incorpore ou utilise un logiciel, ou un logiciel qui utilise, lorsqu'il tourne sur un appareil commandé par un ordinateur approprié, et lorsque l'on a saisi la quantité de cations, d'anions et d'acides aminés contenant du soufre choisis dans un aliment destinée à la consommation animale, la formule 1 de la revendication 1, ou la formule 2 de la revendication 2, ou la formule 3 de la revendication 3, qui équivaut à la quantité de tels cations, anions et acides aminés contenant du soufre par rapport au pH de l'urine, suivant un procédé de prévision du pH de l'urine, dans lequel l'animal est un félin.

**6.** Dispositif selon la revendication 4 ou 5, dans lequel le calculateur ou l'ordinateur ou le site Internet ou le logiciel, valide la saisie d'un utilisateur, comprenant la quantité de sodium, de potassium, de calcium, de magnésium, de chlorure, de soufre, de phosphore, de méthionine, de cystine dans un aliment destinée à la consommation animale, et utilise la totalité ou un sous-ensemble de la saisie, et une ou plusieurs de la formule 1 de la revendication 1, de la formule 2 de la revendication 2, et de la formule 3 de la revendication 3, pour prévoir le pH de l'urine de l'animal qui consomme l'aliment.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 69831105 P
- US 5339771 A
- US 5419283 A

### Non-patent literature cited in the description

- **MARKWELL et al.** *The Journal of Nutrition,* 1998, vol. 128, 2753S-2575S
- **KIENZLE et al.** *Dtsch. tierarzh. wschr.,* 1993, vol. 100, 169-208
- **KIENZLE et al.** *The journal of Nutrition,* 1994, vol. 124, 20652S-2659S
- Nutrient Requirements of Swine. Nat'l Academy Press, 1998
- Nutrient Requirements of Poultry. Nat'l Academy Press, 1994
- Nutrient Requirements of Horses. Nat'l Academy Press, 1989